# EUROPÄISCHE PATENTSCHRIFT

(11) **EP 2 769 707 B1**
(45) Veröffentlichungstag und Bekanntmachung des Hinweises auf die Patenterteilung: **12.04.2017**
(21) Anmeldenummer: 14150915.8
(22) Anmeldetag: 13.01.2014
(51) Int. Cl.: A61K 8/36, A61K 8/39, A61K 8/37

(54) **Wasser-in-Öl-Emulsionen mit mehrschichtigen Pigmentkapseln**
Water-oil emulsions with multilayer pigment capsules
Émulsions eau dans huile avec capsules de pigments multicouches

(30) Priorität: 13.02.2013 DE 102013202329
(43) Veröffentlichungstag der Anmeldung: 27.08.2014
(73) Patentinhaber: Henkel AG & Co. KGaA, 40589 Düsseldorf (DE)
(72) Erfinder: Dickhof, Susanne, 41748 Viersen (DE); Keßler-Becker, Daniela, 51371 Leverkusen (DE)

(56) Entgegenhaltungen:
- EP-A1- 1 477 159
- WO-A1-2004/055119
- WO-A1-2013/106999
- WO-A1-2013/107000
- US-A1- 2009 155 321
- US-A1- 2009 155 322

## Beschreibung

Die Erfindung betrifft Emulsionen, die eine "überraschende" Farbveränderung auf der Haut bewirken können.

Neben der Pflege und Glättung der Haut sowie dem Vorbeugen vor oder der Behandlung von Alterserscheinungen erwarten Verbraucher heutzutage zusätzliche Effekte von kosmetischen Zusammensetzungen. Ein solcher Effekt ist beispielsweise die Tönung bzw. temporäre Farbveränderung der Haut, die nach Möglichkeit nicht okklusiver Natur sein soll, wie es beispielsweise bei abdeckenden Schminken der Fall ist.

So wird zum Beispiel eine gebräunte Haut in der heutigen Zeit vom Verbraucher als Zeichen von Gesundheit und einem aktiven, sportlichen Lebensstil wahrgenommen. Eine braune Haut gilt als attraktiv und der Besitz einer leichten Hautbräune als äußerst erstrebenswert. Seit der im Jahre 1970 erstmalig beobachteten Abnahme der Ozonschicht wird jedoch auch dem Verbraucher immer mehr bewusst, dass der durch intensives Sonnenbaden hervorgerufene Bräunungsprozess mit vielfältigen Schädigungen der Haut einhergeht, welche von vorzeitiger Hautalterung bis zu einem erhöhten Hautkrebsrisiko reichen können. Mit der Anwendung von Bräunungscremes kann der Verbraucher sich den Wunsch nach einer gebräunten Haut erfüllen, ohne sich gleichzeitig verstärkt dem schädlichen UV-A und UV-B Anteil des Sonnenlichtes aussetzen zu müssen.

Die auf dem Markt befindlichen Bräunungscremes enthalten in den meisten Fällen den Wirkstoff 1,3-Dihydroxyaceton. Der Einsatz von dihydroxyacetonhaltigen Cremes kann für den Verbraucher jedoch mit Nachteilen verbunden sein. Einige Konsumenten reagieren auf Dihydroxyaceton allergisch. Der bestehende Verdacht, dass Dihydroxyaceton im Laufe der Zeit Formaldehyd abspalten könnte, lässt den Einsatz von Dihydroxyaceton in Kosmetika ebenfalls als wenig vorteilhaft erscheinen. Weiterhin hat Dihydroxyaceton den Nachteil, dass es zu Geruchsbelästigungen und zu einer ungleichmäßigen Anfärbung der Haut führen kann.

Zudem ist die Hautbräunung mit Substanzen, welche die Haut nachhaltig färben, für einige Verbraucher unerwünscht, da sie nur einzelne Bereiche ihrer Haut oder nur temporär die Hautfarbe variieren möchten.

Cremes, die mit (später leicht abwaschbaren) Pigmenten tönen, sind im Stand der Technik bekannt und erfreuen sich großer Beliebtheit als "leichtes make-up", welches gleichzeitig pflegt und nicht okklusiv wirkt. Allerdings wird das optische Erscheinungsbild dieser Cremes durch die in ihnen enthaltenen Pigmente geprägt, und manche Verbraucher lehnen es ab, ein beispielsweise hell- bis dunkelbraunes Produkt aufzutragen.

Zur Lösung dieses Problems sind Pigment- bzw. Farbstoffzubereitungen entwickelt worden, welche nach außen hell bis weiß wirken, im Inneren aber farbige Pigmente und/oder Farbstoffe beinhalten. Auf diese Weise lassen sich "magische" Cremes herstellen, die als Produkt hell bis weiß aussehen, beim Verstreichen während der Applikation aber eine sichtbare Tönung der Haut bewirken. Entsprechende Pigment- bzw. Farbstoffzubereitungen, oft als sogenannte Kapseln konfektioniert, sind im Markt breit verfügbar. Dennoch besteht auch hier weiterer Verbesserungsbedarf, da insbesondere bei den oft gewünschten braunen Tönungsstufen die zu applizierenden Produkte, insbesondere Emulsionen oft "schmutzig weiß" aussehen. Ein reinweißes Aussehen führt zu größerer Produktakzeptanz und einem stärkeren "Überraschungseffekt", wenn die Produkte bei Applikation die Haut tönen.

Ein weiteres Problem der Tönungscremes ist bislang unzureichend gelöst worden: Bei nicht vollständig homogener Applikation kann sich ein "fleckiges" Hautbild ergeben, in dem einige Bereiche intensiver gefärbt sind als andere, zudem ist ein schnelles Einziehen der Creme wichtig, um eine abriebfeste Tönung zu erreichen.

Die US 2009/155322 A1 offenbart "beads", welche eine pigmenthaltige Matrix enthalten, auf ein Coating aufgebracht worden ist, um ggf. auftretende Zersetzungsprodukte für den Konsumenten unsichtbar zu machen. In der US 2009/155321 A1 werden ähnliche "beads" offenbart.

Die WO 2004/055119 A1 offenbart Interferenzpigmente auf Basis von beschichteten plättchenförmigen Substraten.

In der WO 2013/106999 A1 werden alkoholhaltige Zusammensetzungen offenbart, die ihre Farbe bei der Anwendung verändern. Die Farbveränderung resultiert dabei durch die Freisetzung von Farbstoffen aus Mikrokapseln. Ähnliche Zusammensetzungen werden in der WO 2013/107000 A1 offenbart.

Aufgabe der vorliegenden Erfindung ist es daher, hautpflegende Mittel bereitzustellen, welche die Haut in einem schönen, gleichmäßigen Farbton zu färben vermögen, ohne das Risiko "fleckiger" Tönungen eingehen zu müssen. Darüber hinaus sollte die Abriebfestigkeit der Hauttönung "schmusefest" sein, um Transfer von Farbe z.B. bei Umarmungen zu vermeiden. Begleitend sollten möglichst reinweiße Produkte bereitgestellt werden, um dem Verbraucher die Applikation unangenehm gefärbter "schmutziger" zu ersparen.

Es wurde nun gefunden, dass Öl-in-Wasser-Emulsionen mit bestimmten Pigmentkapseln besonders hell formuliert werden können, auch wenn dunkle Pigmente eingesetzt werden. Die reinweißen Emulsionen sind zudem stabil und ermöglichen eine hoch gleichmäßige Applikation, die zu einem ebenmäßigen Hautbild führt. Darüber hinaus ziehen diese Emulsionen hervorragende ein und bewirken eine schnell abriebfeste Tönung der behandelten Hautareale.

Gegenstand der vorliegenden Erfindung ist in einer ersten Ausführungsform eine Wasser-in-ÖI-Emulsion, enthaltend - bezogen auf ihr Gewicht -
a) 0,1 bis 5 Gew.-% Polyglyceryl-2-dipolyhydroxystearat;
b) mindestens eine Lipid- oder Wachskomponente mit einem Schmelzpunkt > 50°C,
c) mindestens ein unter Normalbedingungen (20°C und 1013,25 mbar) flüssiges Öl, das keinen Riechstoff und kein ätherisches Öl darstellt,
d) 5 bis 70 Gew.-% Wasser, bezogen auf die Gesamtzusammensetzung,
e) mindestens eine mehrschichtige Pigmentkapsel, umfassend
   i) einen Partikelkern,
   ii) eine diesen Partikelkern umgebende erste Hülle, welche - bezogen auf ihr Gewicht - mindestens 50 Gew.-% farbige Pigmente enthält,
   iii) eine die erste Hülle umgebende zweite Hülle, welche - bezogen auf ihr Gewicht - mindestens 50 Gew.-% Titandioxid enthält,
   iv) eine die zweite Hülle umgebende dritte Hülle aus einem wasserunlöslichen, thermoplastischem Polymer.

Soweit nicht anders erwähnt, beziehen sich Mengenangaben im Rahmen der vorliegenden Anmeldung immer auf die gesamte Zusammensetzung, physikalische Größen werden bei 20°C und 1013,25 mbar (= "Normalbedingungen") gemessen.

Als ersten wesentlichen Inhaltsstoff enthalten die erfindungsgemäßen Zusammensetzungen 0,1 bis 5 Gew.-% Polyglyceryl-2-dipolyhydroxystearat. Bei dieser Verbindung handelt es sich um das Homopolymer der 12-Hydroxy-octadecansäure, verestert mit Oxybis(propandiol), das als Wasser-in-Öl-Emulgator wirkt. Im Handel ist Polyglyceryl-2-dipolyhydroxystearat beispielsweise unter der Bezeichnung Dehymuls^{®} PGPH erhältlich.

Erfindungsgemäß bevorzugte Wasser-in-Öl-Emulsionen enthalten 0,25 bis 4 Gew.-%, vorzugsweise 0,5 bis 3 Gew.-%, weiter bevorzugt 0,75 bis 2 Gew.-% und insbesondere 1 bis 1,5 Gew.-% Polyglyceryl-2-dipolyhydroxystearat.

Als weiteren Inhaltsstoff können die erfindungsgemäßen Zusammensetzungen 0,1 bis 10 Gew.-% Polyglyceryl-3-diisostearat enthalten. Im Handel ist Polyglyceryl-3-diisostearat, das als Wasser-in-ÖI-Emulgator wirkt, beispielsweise unter den Bezeichnungen AEC^{®} Polyglyceryl-3 Diisostearate (A & E Connock Ltd.), Cithrol^{®} PG32IS (Croda Europe, Ltd.), Dub^{®} ISO G3 (Stearinerie Dubois Fils), Lameform^{®} TGI (Cognis GmbH) oder Plurol^{®} Diisostearique (Gattefosse s.a.s.) erhältlich. Erfindungsgemäß bevorzugte Wasser-in-ÖI- Emulsionen enthalten 0,5 bis 8 Gew.-%, vorzugsweise 1 bis 5 Gew.-%, weiter bevorzugt 1,5 bis 3 Gew.-% und insbesondere 1 bis 2,5 Gew.-% Polyglyceryl-3-diisostearat.

Als weiteren Inhaltsstoff können die erfindungsgemäßen Zusammensetzungen 0,1 bis 5 Gew.-% Bis-Diglyceryl-Polyacyladipat-2 enthalten. Bei dieser Verbindung handelt es sich um den Adipinsäure-Diester eines gemischten Diglycerylesters von Capryl-, Caprin-, Stearin-, Isostearin- und Hydroxystearinsäure. Im Handel ist Bis-Diglyceryl-Polyacyladipat-2 beispielsweise unter der Bezeichnung Softisan^{®} 649 (Sasol) erhältlich.

Erfindungsgemäß bevorzugte Wasser-in-Ol-Emulsionen enthalten 0,25 bis 4 Gew.-%, vorzugsweise 0,5 bis 3 Gew.-%, weiter bevorzugt 0,75 bis 2 Gew.-% und insbesondere 1 bis 1,5 Gew.-% Bis-Diglyceryl-Polyacyladipat-2.

Als weiteren Inhaltsstoff, der mit den drei vorstehend genannten Inhaltsstoffen hervorragend verträglich ist, können die erfindungsgemäßen Mittel Glycerinmonooleat enthalten. Bevorzugte erfindungsgemäße Wasser-in-ÖI- Emulsionen enthalten zusätzlich, jeweils bezogen auf ihr Gewicht, 0,1 bis 5 Gew.-%, vorzugsweise 0,25 bis 4 Gew.-%, weiter bevorzugt 0,5 bis 3 Gew.-%, noch weiter bevorzugt 0,75 bis 2 Gew.-% und insbesondere 0,9 bis 1,5 Gew.-% Glycerinmonooleat.

Zusätzlich zu den genannten Emulgatoren können die erfindungsgemäßen Mittel optional weitere Emulgatoren aus der Gruppe der Öl-in-Wasser-Emulgatoren und/oder der Wasser-in-Öl-Emulgatoren enthalten.

Bei Öl-in-Wasser-Emulgatoren handelt es sich um dem Fachmann allgemein bekannte Emulgatoren, wie sie beispielsweise in Kirk-Othmer, "Encyclopedia of Chemical Technology", 3. Auflage, 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bevorzugte nichtionische Öl-in-Wasser-Emulgatoren sind ausgewählt aus ethoxylierten C₈-C₂₄-Alkanolen mit durchschnittlich 5 - 100 Mol Ethylenoxid pro Mol, ethoxylierten C₈-C₂₄-Carbonsäuren mit durchschnittlich 5 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 5 - 100 Mol, vorzugsweise 10 - 30 Mol, Ethylenoxid an technische Fettalkohole mit 12 - 18 Kohlenstoffatomen, wie beispielsweise Kokos-, Palm-, Palmkern- oder Talgfettalkohol, sind geeignet.

Besonders bevorzugte Öl-in-Wasser-Emulgatoren sind ausgewählt aus der Gruppe Ceteth-12, Ceteth-20, Ceteth-30, Steareth-12, Steareth-20, Steareth-30, Laureth-12 und Beheneth-20, sowie Mischungen hiervon.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 5 - 100 Mol, vorzugsweise 10 - 30 Mol, Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen.

Weiterhin werden vorzugsweise C₈ - C₂₂-Alkylmono- und -oligoglycoside eingesetzt. C₈ -C₂₂-Alkyl-mono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, die sich von den zwingenden Komponenten a), b) und c) unterscheiden. Besonders bevorzugt sind hier Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Als zusätzliche Wasser-in-Öl-Emulgatoren bevorzugt sind beispielsweise:
- Ester und insbesondere Partialester aus einem Polyol mit 2 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Methylglucosemono- und -diester von linearen, gesättigten und ungesättigten C₁₂ - C₃₀-Carbonsäuren, die hydroxyliert sein können; die Pentaerythritylmono-, -di-, -tri-und -tetraester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-Öl-Emulgatoren mit einem HLB-Wert größer 1,0 und kleiner/ gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon.
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind,
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/ gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen,
- sowie Mischungen der vorgenannten Substanzen.

Erfindungsgemäß außerordentlich bevorzugte zusätzliche Wasser-in-Öl-Emulgatoren sind ausgewählt aus den Mono- und Diestern von Ethylenglycol und den Mono-, Di-, Tri- und Tetraestern von Pentaerythrit mit linearen gesättigten Fettsäuren mit 12 - 30, insbesondere 14 - 22 Kohlenstoffatomen, die hydroxyliert sein können, sowie Mischungen hiervon, die zum Beispiel als Handelsprodukte Cutina PES (INCI: Pentaerythrityl distearate), Cutina AGS (INCI: Glycol distearate) oder Cutina EGMS (INCI: Glycol stearate) erhältlich sind. Diese Handelsprodukte stellen bereits Mischungen aus Mono- und Diestern (bei den Pentaerythritylestern sind auch Tri- und Tetraester enthalten) dar. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Weitere erfindungsgemäß bevorzugte Wasser-in-Öl-Emulgatoren sind ausgewählt aus Stearylalkohol, Cetylalkohol, Glycerylmonostearat, insbesondere in Form der Handelsprodukte Cutina^{®} GMS und Cutina^{®} MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Tri-glycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.

Als zweiten wesentlichen Inhaltsstoff enthalten die erfindungsgemäßen Zusammensetzungen mindestens eine Lipid- oder Wachskomponente mit einem Schmelzpunkt > 50°C. Bevorzugt hat die mindestens eine Lipid- oder Wachskomponente einen Schmelzpunkt im Bereich von 52 -125 °C, besonders bevorzugt im Bereich von 58 - 92 °C.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs und Schellackwachs. Im Sinne der vorliegenden Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Paraffinwachs, Polyalkylenwachse und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren.

Besonders bevorzugt sind Bienenwachs und Paraffinwachs sowie Mischungen aus Bienenwachs und Paraffinwachs. Außerordentlich bevorzugt sind Mischungen aus Bienenwachs und Paraffinwachs im Gewichtsverhältnis von 0,5 - 2, bevorzugt 0,8 - 1,5, besonders bevorzugt 0,9 - 1.

Eine weitere bevorzugte Wachskomponente ist ausgewählt aus mindestens einem Ester aus einem gesättigten, einwertigen C₁₂-C₆₀-Alkanol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkanolen haben sich für die erfindungsgemäße Zusammensetzung als besonders vorteilhaft erwiesen. Die Ester setzen sich aus gesättigten verzweigten oder unverzweigten Monocarbonsäuren und gesättigten verzweigten oder unverzweigten einwertigen Alkanolen zusammen. Auch Ester aus aromatischen Carbonsäuren bzw. Hydroxycarbonsäuren (z. B. 12-Hydroxystearinsäure) und gesättigten verzweigten oder unverzweigten Alkanolen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkanolen einer Kettenlänge von 12 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H und Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C).

Erfindungsgemäß ist als Wachskomponente hydriertes Rizinusöl, erhältlich z.B. als Handelsprodukt Cutina^{®} HR, bevorzugt.

Weitere bevorzugte Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Mischung aus Palmitin- und Stearinsäure).

Die erfindungsgemäßen Mittel können mit besonderem Vorzug Zinkstearat enthalten. Dieses ist eine Lipidkomponente mit einem Schmelzpunkt im Bereich von 120-123°C, also über 50°C. Es fungiert sowohl als Konsistenzgeber und trägt zur einer angenehmeren Produkthaptik bei als auch als Strukturans, das die Produktstabilität verbessert. Erfindungsgemäß bevorzugte Wasser-in-ÖI-Emulsionen sind dadurch gekennzeichnet, dass sie sie 0,25 bis 5 Gew.-%, vorzugsweise 0,5 bis 4 Gew.-%, weiter bevorzugt 0,75 bis 3 Gew.-% und insbesondere 1 bis 2 Gew.-% Zinkstearat enthalten. Außerordentlich bevorzugt sind Mischungen aus Zinkstearat, Bienenwachs und Paraffinwachs, wobei Bienenwachs und Paraffinwachs im Gewichtsverhältnis von 0,5 - 2, bevorzugt 0,8 - 1,5, besonders bevorzugt 0,9 - 1, vorliegen.

Bevorzugte erfindungsgemäße Wasser-in-Öl-Emulsionen sind dadurch gekennzeichnet, dass die Lipid- oder Wachskomponente(n) mit einem Schmelzpunkt > 50°C ausgewählt ist/sind aus Estern aus einem gesättigten, einwertigen C₁₂-C₆₀-Alkanol und einer gesättigten C₈-C₃₆-Monocarbonsäure, insbesondere Lauryllaurat, Laurylmyristat, Laurylpalmitat, Laurylstearat, Lauryl-12-hydroxystearat, Lauryleicosanat, Laurylbehenat, Lauryllignocerat, Laurylcerat, Laurylmyricat, Myristyllaurat, Myristylmyristat, Myristylpalmitat, Myristylstearat, Myristyl-12-hydroxystearat, Myristyleicosanat, Myristylbehenat, Myristyllignocerat, Myristylcerat, Myristylmyricat, Cetyllaurat, Cetylmyristat, Cetylpalmitat, Cetylstearat, Cetyl-12-hydroxystearat, Cetyleicosanat, Cetylbehenat, Cetyllignocerat, Cetylcerat, Cetylmyricat, Stearyllaurat, Stearylmyristat, Stearylpalmitat, Stearylstearat, Stearyl-12-hydroxystearat, Stearyleicosanat, Stearylbehenat, Stearyllignocerat, Stearylcerat, Stearylmyricat, 12-Hydroxystearyllaurat, 12-Hydroxystearylmyristat, 12-Hydroxystearylpalmitat, 12-Hydroxystearylstearat, 12-Hydroxystearyl-12-hydroxystearat, 12-Hydroxystearyleicosanat, 12-Hydroxystearylbehenat, 12-Hydroxystearyllignocerat, 12-Hydroxystearylcerat, 12-Hydroxystearylmyricat, Arachyllaurat, Arachylmyristat, Arachylpalmitat, Arachylstearat, Arachyl-12-hydroxystearat, Arachyleicosanat, Arachylbehenat, Arachyllignocerat, Arachylcerat, Arachylmyricat, Behenyllaurat, Behenylmyristat, Behenylpalmitat, Behenylstearat, Behenyl-12-hydroxystearat, Behenyleicosanat, Behenylbehenat, Behenyllignocerat, Behenylcerat, Behenylmyricat, Lignoceryllaurat, Lignocerylmyristat, Lignocerylpalmitat, Lignocerylstearat, Lignoceryl-12-hydroxystearat, Lignoceryleicosanat, Lignocerylbehenat, Lignoceryllignocerat, Lignocerylcerat, Lignocerylmyricat, Ceryllaurat, Cerylmyristat, Cerylpalmitat, Cerylstearat, Ceryl-12-hydroxystearat, Ceryleicosanat, Cerylbehenat, Ceryllignocerat, Cerylcerat, Cerylmyricat, Myricyllaurat, Myricylmyristat, Myricylpalmitat, Myricylstearat, Myricyl-12-hydroxystearat, Myricyleicosanat, Myricylbehenat, Myricyllignocerat, Myricylcerat, Myricylmyricat, besonders bevorzugt Cetylbehenat, Stearylbehenat und C₂₀-C₄₀-Alkylstearate, insbesondere Arachylstearat, Behenylstearat, Lignocerylstearat, Cerylstearat und Myricylstearat, weiterhin ausgewählt aus Glycerintriestern von gesättigten linearen C₁₂ - C₃₀-Carbonsäuren, die hydroxyliert sein können, wobei diese Glycerintriester bevorzugt in Form natürlicher Wachse, insbesondere Candelillawachs, Carnaubawachs oder Bienenwachs, oder bevorzugt in Form natürlicher Öle, die vollständig hydriert (gehärtet) sind, vorliegen können, insbesondere vollständig hydriertes gehärtetes Rizinusöl (Tri-12-hydroxystearin), Tristearin, Tribehenin, vollständig hydriertes Sojabohnenöl, vollständig hydriertes Maiskeimöl, vollständig hydriertes Sonnenblumenöl, vollständig hydriertes Erucasäurereiches Rapsöl (HEAR Öl), vollständig hydriertes Erucasäure-armes Rapsöl (LEAR Öl), vollständig hydriertes Canolaöl, vollständig hydriertes Crambe-Öl, vollständig hydriertes Wiesenschaumkrautöl, vollständig hydriertes Baumwollsamenöl, vollständig hydriertes Olivenöl, vollständig hydriertes Distelöl, vollständig hydriertes Sonnenblumenöl, vollständig hydriertes Sesamöl, vollständig hydriertes Kokosöl, vollständig hydriertes Palmöl, vollständig hydriertes Palmkernöl, vollständig hydriertes Babassuöl, vollständig hydriertes Erdnussöl, vollständig hydrierte Kakaobutter, Sheabutter, Illipebutter, gehärtete tierische Fette, insbesondere Talg oder Speck, vollständig hydrierte Öle marinen Ursprungs, wie Schwertfischöl, Sardinenöl, Walöl und Heringsöl.

Weitere bevorzugte erfindungsgemäße Wasser-in-Öl-Emulsionen sind dadurch gekennzeichnet, dass die Lipid- oder Wachskomponente/n d) in einer Gesamtmenge von 1 - 10 Gew.-%, bevorzugt 1,5 - 8 Gew.-%, besonders bevorzugt 2 - 6 Gew.-%, außerordentlich bevorzugt 2,5 - 4 Gew.-%, jeweils bezogen auf die Gesamtzusammensetzung, enthalten ist. In einer besonders bevorzugten Ausführungsform ist Bienenwachs in einer Gesamtmenge von 0,1 - 5 Gew.-%, bevorzugt 0,5 - 3 Gew.-%, besonders bevorzugt 0,8 - 1,5 Gew.-%, jeweils bezogen auf die Gesamtzusammensetzung, enthalten. Als dritten wesentlichen Inhaltsstoff enthalten die erfindungsgemäßen Zusammensetzungen mindestens ein unter Normalbedingungen flüssiges Öl, das keinen Riechstoff und kein ätherisches Öl darstellt.

Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Bevorzugte erfindungsgemäße Wasser-in-Öl-Emulsionen enthalten 2,5 bis 20 Gew.-%, vorzugsweise 5 bis 17,5 Gew.-%, weiter bevorzugt 7,5 bis 15 und insbesondere 10 bis 12,5 Gew.-% unter Normalbedingungen flüssige(s) Öl(e), das/die keinen Riechstoff und kein ätherisches Öl darstellt/darstellen.

Das erfindungsgemäß in den Emulsionen enthaltene unter Normalbedingungen flüssiges Öl stellt weder einen Riechstoff noch ein ätherisches Öl dar.

Erfindungsgemäß bevorzugte Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16), Octyldodecanol (Eutanol^{®} G) und 2-Ethylhexylalkohol.

Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat).

Weitere erfindungsgemäß bevorzugte Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maiskeimöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Bevorzugte erfindungsgemäße Wasser-in-ÖI- Emulsionen sind dadurch gekennzeichnet, dass das unter Normalbedingungen flüssige Öl e) ausgewählt ist aus Benzoesäureestern von linearen oder verzweigten C₈-C₂₂-Alkanolen, verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, sofern diese Triglyceride unter Normalbedingungen flüssig sind, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, sofern diese Ester unter Normalbedingungen flüssig sind, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.

Ein erfindungsgemäß bevorzugter Ester von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, sofern diese Ester unter Normalbedingungen flüssig sind, ist Cetearylisononanoat.

Es kann erfindungsgemäß außerordentlich bevorzugt sein, Mischungen der vorgenannten Öle einzusetzen. Besonders bevorzugt sind Mischungen aus Benzoesäure-C12-C15-alkylestern, Capric/Caprylic Triglycerides und Cetearylisononanoat.

Wie bereits in der Aufgabenstellung erwähnt, können die erfindungsgemäßen Zusammensetzungen mit deutlich reduziertem Gehalt an mineralölbasierten Verbindungen hergestellt werden. Es ist erfindungsgemäß bevorzugt, auf diese Verbindungen weitgehend zu verzichten.

Entsprechende weniger geeignete oder (je nach verwendetem Wasser-in-Öl-Emulgator) sogar ungeeignete Ölkomponenten sind zum Beispiel Siliconöle und Kohlenwasserstofföle.

Siliconöle, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen zu diesen erfindungsgemäß weniger bevorzugten Ölkomponenten.

Natürliche und synthetische Kohlenwasserstoffe wie beispielsweise Paraffinöle, Isohexadecan, Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S) gehören ebenfalls zu den erfindungsgemäß weniger bevorzugten Ölkomponenten.

Der Anteil der Siliconöle und/oder Kohlenwasserstoffe sollte daher in einer bevorzugten Ausführungsform der Erfindung nicht größer sein als 10 %, bezogen auf das Gesamtgewicht an unter Normalbedingungen flüssigen Ölen. In einer besonders bevorzugten Ausführungsform der Erfindung sind keine Siliconöle und/oder Kohlenwasserstoffe, insbesondere keine Paraffin- und Isoparaffin-Kohlenwasserstoffe, enthalten.

Bevorzugte erfindungsgemäße Wasser-in-ÖI- Emulsionen sind dadurch gekennzeichnet, dass sie sie weniger als 5 Gew.-%, vorzugsweise weniger als 4 Gew.-%, weiter bevorzugt weniger als 3 Gew.-%, noch weiter bevorzugt weniger als 2 Gew.-% und insbesondere weniger als 1 Gew.-% mineralische Öle und Wachse enthalten.

Weiter bevorzugte erfindungsgemäße Wasser-in-ÖI- Emulsionen sind dadurch gekennzeichnet, dass sie sie weniger als 5 Gew.-%, vorzugsweise weniger als 4 Gew.-%, weiter bevorzugt weniger als 3 Gew.-%, noch weiter bevorzugt weniger als 2 Gew.-% und insbesondere weniger als 1 Gew.-% siliconhaltige Verbindungen enthalten.

Die erfindungsgemäßen Zusammensetzungen enthalten mindestens eine mehrschichtige Pigmentkapsel, die einen Partikelkern, eine diesen Partikelkern umgebende erste Hülle, welche - bezogen auf ihr Gewicht - mindestens 50 Gew.-% farbige Pigmente enthält, eine die erste Hülle umgebende zweite Hülle, welche - bezogen auf ihr Gewicht - mindestens 50 Gew.-% Titandioxid enthält und eine die zweite Hülle umgebende dritte Hülle aus einem wasserunlöslichen, thermoplastischem Polymer umfasst. Nach außen ist diese Kapsel durch das wasserunlösliche, thermoplastische Polymer abgeschlossen und kann sowohl in wässrigen als auch in ölbasierten kosmetischen Systemen eingesetzt werden, ohne dass ein Ausbluten oder eine mangelnde Lagerstabilität zu befürchten wäre. Erst durch mechanische Einwirkung bei der Applikation des Kosmetikums "zerbricht" die Kapsel und setzt die Pigmente aus den inneren Hüllschichten frei.

Das optische Erscheinungsbild der Kapseln wird durch die unter der äußeren Hülle befindliche zweite Hülle geprägt. Durch den hohen Gehalt an Titandioxid in dieser Hülle wirken die Kapseln nach außen hell bis weiß, auch wenn in der ersten, direkt auf dem Partikelkern befindlichen Schicht dunkle Pigmente enthalten sind.

Das Kapselinnere wird durch einen Partikelkern gebildet. Als Partikelkern kommen grundsätzlich alle bei Raumtemperatur (25°C) festen Substanzen in Frage, im Hinblick auf die rückstandsfreie Applikation sind wasserlösliche Substanzen bevorzugt. Besonders bevorzugte Trägermaterialien sind ausgewählt aus Zuckern und Zuckeralkoholen, unter diesen sind die Zuckeralkohole besonders bevorzugt. Besonders bevorzugte Alditole sind Mannit (Mannitol), Isomalt, Lactit, Sorbit (Sorbitol oder Glucitol) und Xylit (Xylitol), Threit, Erythrit und Arabit.

Erfindungsgemäß bevorzugte Wasser-in-Öl-Emulsionen sind dadurch gekennzeichnet, dass der Partikelkern der mehrschichtigen Pigmentkapsel
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% seines Gewichts aus

Alditolen, vorzugsweise aus Mannitol, besteht.

Ganz besonders bevorzugte erfindungsgemäße Wasser-in-Öl-Emulsionen enthalten mehrschichtigen Pigmentkapseln, welche - bezogen auf das Gewicht der Pigmentkapsel - 10 bis 40 Gew.-%, vorzugsweise 12,5 bis 37,5 Gew.-%, weiter bevorzugt 15 bis 35 Gew.-%, noch weiter bevorzugt 17,5 bis 32,5 Gew.-%, besonders bevorzugt 20 bis 30 Gew.-% und insbesondere 22,5 bis 27,5 Gew.-% Mannitol enthalten.

Die erfindungsgemäßen Zusammensetzungen ergeben bei Applikation überraschende Effekte, indem eine nach außen helle bis weiße Zusammensetzung einen dunkleren Applikationserfolg herbeiführt. Dies ist die Folge des Kollabierens der mehrschichtigen Pigmentkapseln, wobei die von außen durch die dritte und zweite Hülle zunächst nicht sichtbaren farbigen Pigmente der ersten Hülle freigesetzt werden. Die den Partikelkern umgebende erste Hülle, enthält - bezogen auf ihr Gewicht - mindestens 50 Gew.-% farbige Pigmente.

Bevorzugte Pigmente dieser Art können anorganisch oder organisch sein. Weitere bevorzugte Pigmente weisen einen zahlenmittleren Teilchendurchmesser von 0,1 - 200 µm, bevorzugt 0,5 - 100 µm, besonders bevorzugt 1 - 50 µm und außerordentlich bevorzugt 2 - 30 µm auf.

Die Farbpigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Besonders bevorzugte Farbpigmente sind beispielsweise Eisenoxide (z. B. Fe₂O₃, Fe₃O₄, FeO(OH)) und/oder Zinnoxid.

Einsetzbare farbige Pigmente sind beispielsweise Pigment Green (CI 10006, grün), Pigment Yellow 1 (CI 11680, gelb), Pigment Yellow 3 (CI 11710, gelb), Pigment Orange 1 (CI 11725, orange), Pigment Red 3 (CI 12120, rot), Pigment Red 112 (CI 12370, rot), Pigment Red 7 (CI 12420, rot), Pigment Brown 1 (CI 12480, braun), Pigment Yellow 13 (CI 21100, gelb), Pigment Yellow 83 (CI 21108, gelb), Pigment Violet 23 (CI 51319, violett), Pigment Red 122 (CI 73915, rot), Pigment Blue 16 (CI 74100, blau), Pigment Red 101 und 102 (CI 77015, rot), Kohlenstoff (CI 77266, schwarz), Pigment Black 9 (CI 77267, schwarz), Carbo medicinalis vegetabilis (CI 77268:1, schwarz), Chromoxid (CI 77288, grün), Chromoxid, wasserhaltig (CI 77289, grün), Pigment Blue 28, Pigment Green 14 (CI 77346, grün), Pigment Metal 2 (CI 77400, braun), Gold (CI 77480, braun), Eisenoxide und -hydoxide (CI 77489, orange), Eisenoxid (CI 77491, rot), Eisenoxidhydrat (CI 77492, gelb), Eisenoxid (CI 77499, schwarz), Mischungen aus Eisen(II)- und Eisen(III)-hexacyanoferrat (CI 77510, blau), Silber (CI 77820, weiß). Die Wahl der Pigmente in der ersten Hülle kann dem gewünschten Anwendungszweck des Kosmetikums angepaßt werden. Um beispielsweise eine "gesunde Bräune" herbeizuführen, können gelbe, orange oder braune bis schwarze Pigmente oder deren Mischungen eingesetzt werden.

Zur Kaschierung von unerwünschten Hautrötungen werden im Sinne der Komplementärfarblehre vorteilhaft Rotlicht filternde Farbpigmente, insbesondere Grünpigmente, eingesetzt. Aber auch Mischungen können besondere Effekte bei der Teint-Korrektur ergeben. So ist der Zusatz von Blaupigmenten, bevorzugt im Gewichtsverhältnis 1:1 bis 1:100 in Bezug auf das eingesetzte Grünpigment, erfindungsgemäß bevorzugt. Das Blaupigment erhöht die farbverändernde Wirkung, so dass die rötliche Hautfarbe besser in ein natürliches Braun, d.h. eine natürliche Hautfarbe, umgewandelt wird.

Vorteilhafte Grünpigmente können aus der folgenden Gruppe allein oder in Kombination ausgewählt werden: Colorona Brilliant Green, Colorona Egyptian Emerald, Timiron Splendid Green, Vert Oxyde Crome Anhydre N, Vert Oxyde Crome Hydrate W886 und/oder Outremer Supercosmetique W 6803. Diese genannten Pigmente werden u. a. auch unter den entsprechenden CI Nummern gelistet. Bevorzugt sind daher insbesondere die Grünpigmente mit den CI Nummern CI 77288, CI 77289, CI 77007, CI 77891, CI 77491, CI 77499, CI 77891, CI 77499, CI 77288 und CI 77492.

Rotlicht filternd bedeutet hierin, dass der Rotlichtanteil der sichtbaren Lichtes absorbiert wird und die Komplementärfarbe Grün sichtbar wird.

Durch den Auftrag einer erfindungsgemäßen Zubereitung mit Rotlicht filternden Farbstoffen, wie beispielsweise die angegebenen Grünpigmente, auf die Haut, die beispielsweise durch Sonnenbestrahlung oder aufgrund Rosacea rötlich gefärbt erscheint, wird für den Betrachter das Erscheinungsbild der Haut natürlich erscheinen. Um diesen Effekt zu generieren, ist die Auswahl und Menge an Rotlicht filternden Farbstoffen wesentlich.

Erfindungsgemäß bevorzugte Wasser-in-Öl-Emulsionen sind dadurch gekennzeichnet, dass die den Partikelkern umgebende erste Hülle
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% ihres Gewichts aus einem oder mehreren der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) besteht.

Besonders bevorzugte erfindungsgemäße Wasser-in-Öl-Emulsionen enthalten mehrschichtigen Pigmentkapseln, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) enthalten.

Ganz besonders bevorzugte erfindungsgemäße Wasser-in-Öl-Emulsionen enthalten mehrschichtigen Pigmentkapseln, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) enthalten.

Um die überraschenden Effekte bei der Applikation zu gewährleisten, wird in den Pigmentkapseln in den erfindungsgemäßen Zubereitungen die farbige erste Hülle durch eine weiße zweite Hülle "kaschiert", welche - bezogen auf ihr Gewicht - mindestens 50 Gew.-% Titandioxid enthält. Erfindungsgemäß bevorzugte Wasser-in-Öl-Emulsionen sind dadurch gekennzeichnet, dass die die erste Hülle umgebende zweite Hülle
- 20 bis 40 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Titandioxid (CI 77891) besteht.

Besonders bevorzugte erfindungsgemäße Wasser-in-Öl-Emulsionen enthalten mehrschichtigen Pigmentkapseln, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (CI 77891).

Ganz besonders bevorzugte erfindungsgemäße -Wasser-in-Öl-Emulsionen enthalten mehrschichtigen Pigmentkapseln, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% Titandioxid (CI 77891)
enthalten.

Nach außen werden die in den erfindungsgemäßen Zusammensetzungen eingesetzten Pigmentkapseln durch eine dritte Hülle aus einem wasserunlöslichen, thermoplastischem Polymer abgeschlossen. Unter "Polymer" werden dabei im Rahmen der vorliegenden Erfindung sowohl Homoals auch Copolymere als auch Polymergemische verstanden.

Zu den erfindungsgemäß geeigneten Thermoplasten zählen z. B.: Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC).

Im Hinblick auf die optischen Eigenschaften der Pigmentkapseln, auf ihre Verarbeitbarkeit (Herstellung der Kapseln, statische Aufladung und Stauben bei der Verarbeitung etc.) sowie auf ihre Verträglichkeit mit den übrigen Bestandteilen der erfindungsgemäßen Zusammensetzungen hat sich insbesondere Polymethylmethacrylat (PMMA) als besonders geeignetes Material für die dritte Hülle erwiesen. Erfindungsgemäß bevorzugte Wasser-in-Öl-Emulsionen sind dadurch gekennzeichnet, dass die die zweite Hülle umgebende dritte Hülle
- 2 bis 7 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Polymethylmethacrylat besteht.

Die in den erfindungsgemäßen Zusammensetzungen eingesetzten Pigmentkapseln können beliebige Partikelgrößen aufweisen. Größere Kapseln mit Durchmessern bis zu 2 mm können optisch reizvolle Akzente in der Formulierung setzen, haben aber den Nachteil, die farbigen Pigmente bei der Applikation lokal in größerer Menge freizusetzen, so dass für ein einheitliches Anwendungsergebnis ein umfangreicheres Verreiben erforderlich ist. Kleinere Kapseln können in größerer Menge eingesetzt werden und erzielen so bereits mit wenig Applikationsaufwand eine einheitliche und homogene Freisetzung der verkapselten Farbpigmente.

Besonders bevorzugte erfindungsgemäße Wasser-in-Öl-Emulsionen sind dadurch gekennzeichnet, dass mindestens 90 Gew.-% der mehrschichtigen Pigmentkapseln eine Partikelgröße von 75 nm bis 211 µm (70 bis 200 Mesh) aufweisen.

Zusätzlich zu den genannten Inhaltsstoffen können die erfindungsgemäßen Emulsionen weitere Inhaltsstoffe enthalten. Im Folgenden werden bevorzugte Inhaltsstoffe der erfindungsgemäßen Mittel näher beschrieben.

In einer weiteren bevorzugten Ausführungsform enthält die erfindungsgemäßen Emulsionen mindestens eine UV-absorbierende Substanz. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Deaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäurederivate, vorzugsweise 4-(Di-methylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Meth-oxyzimtsäure-2-ethylhexylester, 4-Methoxyzimtsäurepropylester, 4-Methoxyzimtsäureiso-amyl-ester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4'-methylbenzophenon, 2,2'-Dihy-droxy-4-methoxybenzophenon; Ester der Benzalmalonsäure, vorzugsweise 4-Methoxy-benz-malonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zink-stearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Emulsionen in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten.

In einer weiteren bevorzugten Ausführungsform liegen die erfindungsgemäßen Mittel in flüssiger Form vor. Zum Erreichen einer flüssigen Konsistenz kann der Einsatz sowohl flüssiger organischer Lösungsmittel, wie auch der von Wasser angezeigt sein. Bevorzugte erfindungsgemäße Mittel enthalten daher gegebenenfalls mindestens ein Lösungsmittel.

Lösungsmittel, die in bevorzugten erfindungsgemäßen Mitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glycol, Propan- oder Butandiol, Glycerin, Diglyol, Propyl- oder Butyldiglycol, Hexylenglycol, Ethylenglycolmethylether, Ethylenglycolethylether, Ethylen-glycolpropylether, Ethylenglycolmono-n-butylether, Diethylenglycolmethylether, Diethylenglycolethylether, Propylenglycolmethyl-, -ethyl- oder -propyl-ether, Butoxypropoxy-propanol (BPP), Dipropylenglycolmonomethyl-, oder -ethylether, Di-isopropylenglycolmonomethyl-, oder -ethylether, Methoxy-, Ethoxy- oder Butoxytriglycol, 1-Butoxyethoxy-2-propanol, 3-Methyl-3-methoxybutanol, Propylen-glycol-t-butylether sowie Mischungen dieser Lösungsmittel.

Zu den Alkoholen, die in der vorliegenden Erfindung vorzugsweise als Cosolventien eingesetzt werden können, gehören flüssige Polyethylenglycole, mit niederem Molekulargewicht, beispielsweise Polyethylenglycole mit einem Molekulargewicht von 200, 300, 400 oder 600. Weitere geeignete Cosolventien sind andere Alkohole, zum Beispiel (a) niedere Alkohole wie Ethanol, Propanol, Isopropanol und n-Butanol, (b) Ketone wie Aceton und Methylethylketon, (c) C₂-C₄-Polyole wie ein Diol oder ein Triol, beispielsweise Ethylenglycol, Propylenglycol, Glycerin oder Gemische davon. Insbesondere bevorzugt ist aus der Klasse der Diole 1,2-Octandiol.

In einer bevorzugten Ausführungsform enthält das erfindungsgemäße Mittel ein oder mehrere Lösungsmittel aus der Gruppe, umfassend C₁- bis C₄-Monoalkohole, C₂- bis C₆-Glycole, C₃- bis C₁₂-Glycolether und Glycerin, insbesondere Ethanol. Die erfindungsgemäßen C₃- bis C₁₂-Glycolether enthalten Alkyl- bzw. Alkenylgruppen mit weniger als 10 Kohlenstoffatomen, vorzugsweise bis zu 8, insbesondere bis zu 6, besonders bevorzugt 1 bis 4 und äußerst bevorzugt 2 bis 3 Kohlenstoffatomen. Bevorzugte C₁- bis C₄-Monoalkohole sind Ethanol, *n*-Propanol, *iso*-Propanol und *tert*-Butanol. Bevorzugte C₂- bis C₆-Glycole sind Ethylenglycol, 1,2-Propylenglycol, 1,3-Propylenglycol, 1,5-Pentandiol, Neopentylglycol und 1,6-Hexandiol, insbesondere Ethylenglycol und 1,2-Propylenglycol. Bevorzugte C₃- bis C₁₂-Glycolether sind Di-, Tri-, Tetra- und Pentaethylenglycol, Di-, Tri-und Tetrapropylenglycol, Propylenglycolmonotertiärbutylether und Propylenglycolmonoethylether sowie die gemäß INCI bezeichneten Lösungsmittel Butoxydiglycol, Butoxyethanol, Butoxyisopropanol, Butoxypropanol, Butyloctanol, Ethoxydiglycol, Ethoxyethanol, Ethyl Hexanediol, Isobutoxypropanol, Isopentyldiol, 3-Methoxybutanol, Methoxyethanol, Methoxyisopropanol und Methoxymethylbutanol. Bevorzugte erfindungsgemäße Mittel enthalten ein oder mehrere Lösungsmittel aus der Gruppe, umfassend C₁- bis C₄-Monoalkohole, C₂- bis C₆-Glycole, C₃- bis C₁₂-Glycolether und Glycerin, in einer Gesamtmenge von 0,1 bis 30 Gew.-%, insbesondere 2 bis 20 Gew.-%, besonders bevorzugt 3 bis 15 Gew.-%, äußerst bevorzugt 5 bis 12 Gew.-%, beispielsweise 5,3 oder 10,6 Gew.-%, jeweils bezogen auf das gesamte Mittel.

Das erfindungsgemäße Mittel kann vorzugsweise einen oder mehrere antimikrobielle Wirkstoffe bzw. Konservierungsmittel in einer Menge von üblicherweise 0,0001 bis 3 Gew.-%, vorzugsweise 0,0001 bis 2 Gew.-%, insbesondere 0,0002 bis 1 Gew.-%, besonders bevorzugt 0,0002 bis 0,2 Gew.-%, äußerst bevorzugt 0,0003 bis 0,1 Gew.-%, enthalten.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung. Geeignete antimikrobielle Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-di-cyanobutan, lodo-2-propyl-butyl-carbamat, Iod, lodophore, Peroxoverbindungen, Halogenverbindungen sowie beliebigen Gemischen der voranstehenden Substanzen.

Weitere bevorzugte erfindungsgemäße Zusammensetzungen enthalten zur Verbesserung der Hautfeuchtigkeit unter Verzicht auf komedogene Substanzen mindestens ein alkyl- oder hydroxyalkylsubstituierten Harnstoff der allgemeinen Formel (A), 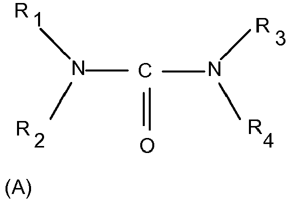 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-HydroxyalkylGruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-HydroxyalkylGruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Bis-N,N'- (2-hydroxyethyl)harnstoff.

Erfindungsgemäß besonders bevorzugte Zusammensetzungen sind dadurch gekennzeichnet, dass sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins). Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt.

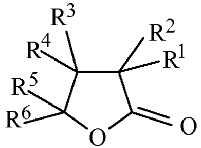

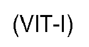

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Amino-benzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.

- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Erfindungsgemäß besonders bevorzugte Zusammensetzungen sind dadurch gekennzeichnet, dass sie mindestens ein Vitamin K in einer Gesamtmenge von 0001 bis 1,0 Gew.-%, bevorzugt 0,05 bis 0,01 Gew.-%, besonders bevorzugt 0,1 bis 0,5 Gew.-%, jeweils bezogen auf die gesamte Zusammensetzung, enthalten.

Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

Ein erfindungsgemäßes Parfümöl kann einzelne Riechstoffverbindungen, z.B. die synthetischen Produkte vom Typ der Ester, Ether, Aldehyde, Ketone, Alkohole und Kohlenwasserstoffe enthalten. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexyl-propionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote des gebildeten Parfümöl erzeugen.

Die Parfümöle können aber auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" (top note), "Herz- bzw. Mittelnote" (middle note bzw. body) sowie "Basisnote" (end note bzw. dry out) unterteilt.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümöl vorteilhaft einsetzbar sind, zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linalylacetat und -propionat, Menthol, Menthon, Methyl-n-hep-tenon, Phellandren, Phenylacetaldehyd, Terpinylacetat, Zitral, Zitronellal.

Alle vorgenannten Riechstoffe sind alleine oder in Mischung in den Parfümölen gemäß der vorliegenden Erfindung mit den bereits genannten Vorteilen einsetzbar.

Insbesondere können auch Duftsstoffe aus der Gruppe der Allylalkoholester, Ester sekundärer Alkohole, Ester tertiärer Alkohole, allylische Ketone, Acetale, Ketale, Kondensationsprodukte von Aminen und Aldehyden und/oder deren Mischungen im Parfümöl enthalten sein.

In einer bevorzugten Ausführungsform enthält das erfindungsgemäße Mittel bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf das gesamte Mittel.

In einer bevorzugten Ausführungsform enthalten die Parfümöle weniger als 8, vorteilhafterweise weniger als 7, in vorteilhafterer Weise weniger als 6, in wiederum vorteilhafterer Weise weniger als 5, in weiter vorteilhafterweise weniger als 4, noch vorteilhafter weniger als 3, vorzugsweise weniger als 2, insbesondere keine Duftstoffe aus der Liste Amylcinnamal, Amylcinnamylalkohol, Benzylalkohol, Benzylsalicylat, Cinnamylalkohol, Cinnamal, Citral, Cumarin, Eugenol, Geraniol, Hydroxycitronellal, Hydroxymethylpentylcyclohexencarboxaldehyd, Isoeugenol, Anisylalkohol, Benzylbenzoat, Benzylcinnamat, Citronellol, Farnesol, Hexylcinnamaldehyd, Lilial, d-Limonen, Linalool, Methylheptincarbonat, 3-Methyl-4-(2,6,6-trimethyl-2-cyclohexen-1-yl)-3-buten-2-on, Eichenmoosextrakt, Baummoosextrakt.

## Patentansprüche

1. Wasser-in-Öl-Emulsion, enthaltend - bezogen auf ihr Gewicht -
a) 0,1 bis 5 Gew.-% Polyglyceryl-2-dipolyhydroxystearat;
b) mindestens eine Lipid- oder Wachskomponente mit einem Schmelzpunkt > 50°C,
c) mindestens ein unter Normalbedingungen (20°C und 1013,25 mbar) flüssiges Öl, das keinen Riechstoff und kein ätherisches Öl darstellt,
d) 5 bis 70 Gew.-% Wasser, bezogen auf die Gesamtzusammensetzung,
e) mindestens eine mehrschichtige Pigmentkapsel, umfassend
i) einen Partikelkern,
ii) eine diesen Partikelkern umgebende erste Hülle, welche - bezogen auf ihr Gewicht - mindestens 50 Gew.-% farbige Pigmente enthält,
iii) eine die erste Hülle umgebende zweite Hülle, welche - bezogen auf ihr Gewicht - mindestens 50 Gew.-% Titandioxid enthält,
iv) eine die zweite Hülle umgebende dritte Hülle aus einem wasserunlöslichen, thermoplastischem Polymer.

2. Wasser-in-Öl-Emulsion nach Anspruch 1, **dadurch gekennzeichnet, dass** sie, bezogen auf ihr Gewicht, 0,25 bis 4 Gew.-%, vorzugsweise 0,5 bis 3 Gew.-%, weiter bevorzugt 0,75 bis 2 Gew.-% und insbesondere 1 bis 1,5 Gew.-% Polyglyceryl-2-dipolyhydroxystearat enthält.

3. Wasser-in-Öl-Emulsion nach einem der Ansprüche 1 oder 2, **dadurch gekennzeichnet, dass** zusätzlich 0,1 bis 5 Gew.-%, vorzugsweise 0,25 bis 4 Gew.-%, weiter bevorzugt 0,5 bis 3 Gew.-%, noch weiter bevorzugt 0,75 bis 2 Gew.-% und insbesondere 0,9 bis 1,5 Gew.-%, jeweils bezogen auf ihr Gewicht, Glycerinmonooleat enthält.

4. Wasser-in-Öl-Emulsion nach einem der Ansprüche 1 bis 3, **dadurch gekennzeichnet, dass** sie, bezogen auf ihr Gewicht, 0,25 bis 5 Gew.-%, vorzugsweise 0,5 bis 4 Gew.-%, weiter bevorzugt 0,75 bis 3 Gew.-% und insbesondere 1 bis 2 Gew.-% Zinkstearat enthält.

5. Wasser-in-Öl-Emulsion nach einem der Ansprüche 1 bis 4, **dadurch gekennzeichnet, dass** der Partikelkern der mehrschichtigen Pigmentkapsel
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% seines Gewichts aus Alditolen, vorzugsweise aus Mannitol, besteht.

6. Wasser-in-Öl-Emulsion nach einem der Ansprüche 1 bis 5, **dadurch gekennzeichnet, dass** sie mehrschichtige Pigmentkapseln enthält, welche - bezogen auf das Gewicht der Pigmentkapsel
- 10 bis 40 Gew.-%, vorzugsweise 12,5 bis 37,5 Gew.-%, weiter bevorzugt 15 bis 35 Gew.-%, noch weiter bevorzugt 17,5 bis 32,5 Gew.-%, besonders bevorzugt 20 bis 30 Gew.-% und insbsondere 22,5 bis 27,5 Gew.-% Mannitol enthalten.

7. Wasser-in-Öl-Emulsion nach einem der Ansprüche 1 bis 6, **dadurch gekennzeichnet, dass** die den Partikelkern umgebende erste Hülle
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% ihres Gewichts aus einem oder mehreren der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) besteht.

8. Wasser-in-Öl-Emulsion nach einem der Ansprüche 1 bis 7, **dadurch gekennzeichnet, dass** sie mehrschichtige Pigmentkapseln enthält, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) enthalten.

9. Wasser-in-Öl-Emulsion nach einem der Ansprüche 1 bis 8, **dadurch gekennzeichnet, dass** die die erste Hülle umgebende zweite Hülle
- 20 bis 40 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Titandioxid (CI 77891) besteht.

10. Wasser-in-01-Emulsion nach einem der Ansprüche 1 bis 9, **dadurch gekennzeichnet, dass** sie mehrschichtige Pigmentkapseln enthält, welche - bezogen auf das Gewicht der Pigmentkapsel
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz)
enthalten.

11. Wasser-in-ÖI-Emulsion nach einem der Ansprüche 1 bis 10, **dadurch gekennzeichnet, dass** die die zweite Hülle umgebende dritte Hülle
- 2 bis 7 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Polymethylmethacrylat besteht.

12. Wasser-in-ÖI-Emulsion nach einem der Ansprüche 1 bis 11, **dadurch gekennzeichnet, dass** sie mehrschichtige Pigmentkapseln enthält, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% Titandioxid (CI 77891) enthalten.

13. Wasser-in-Öl-Emulsion nach einem der Ansprüche 1 bis 12, **dadurch gekennzeichnet, dass** sie mehrschichtige Pigmentkapseln enthält, welche - bezogen auf das Gewicht der Pigmentkapsel
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% eines oder mehrerer der Pigmente Pigment Green (CI 10006 grün), Pigment Brown 1 (CI 12480 braun), Chlorierte Phthalocyanine (CI 74260 grün), Pigment Red 101 und 102 (CI 77015 rot), Kohlenstoff (CI 77266 schwarz), Pigment Black 9 (CI 77267 schwarz), Pigment Blue 28, Pigment Green 14 (CI 77346 grün), Pigment Metal 2 (CI 77400 braun), Gold (CI 77480 braun), Eisenoxide und -hydoxide (CI 77489 orange), Eisenoxid (CI 77491 rot), Eisenoxid (CI 77499 schwarz) und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% Titandioxid (CI 77891)
enthalten.

14. Wasser-in-Öl-Emulsion nach einem der Ansprüche 1 bis 13, **dadurch gekennzeichnet, dass** mindestens 90 Gew.-% der mehrschichtigen Pigmentkapseln eine Partikelgröße von 75 nm bis 211 µm (70 bis 200 Mesh) aufweisen.

## Claims

1. A water-in-oil emulsion containing, based on its weight,
a) from 0.1 to 5 wt.% polyglyceryl-2-dipolyhydroxystearate;
b) at least one lipid or wax component having a melting point of > 50°C,
c) at least one oil that is liquid under normal conditions (20°C and 1013.25 mbar) and that is not an odorant or an essential oil,
d) from 5 to 70 wt.% water, based on the total composition,
e) at least one multilayer pigment capsule comprising
i) a particle core,
ii) a first shell surrounding this particle core that contains, based on its weight, at least 50 wt.% colored pigments,
iii) a second shell surrounding the first shell that contains, based on its weight, at least 50 wt.% titanium dioxide,
iv) a third shell surrounding the second shell made of a water-insoluble, thermoplastic polymer.

2. The water-in-oil emulsion according to claim 1, **characterized in that** it contains, based on its weight, from 0.25 to 4 wt.%, preferably from 0.5 to 3 wt.%, more preferably from 0.75 to 2 wt.% and in particular from 1 to 1.5 wt.% polyglyceryl-2-dipolyhydroxystearate.

3. The water-in-oil emulsion according to either claim 1 or claim 2, **characterized in that** it additionally contains, in each case based on its weight, from 0.1 to 5 wt.%, preferably from 0.25 to 4 wt.%, more preferably from 0.5 to 3 wt.%, even more preferably from 0.75 to 2 wt.% and in particular from 0.9 to 1.5 wt.% glycerol monooleate.

4. The water-in-oil emulsion according to one of claims 1 to 3, **characterized in that** it contains, based on its weight, from 0.25 to 5 wt.%, preferably from 0.5 to 4 wt.%, more preferably from 0.75 to 3 wt.% and in particular from 1 to 2 wt.% zinc stearate.

5. The water-in-oil emulsion according to one of claims 1 to 4, **characterized in that** the particle core of the multilayer pigment capsule
- makes up from 20 to 40 wt.% of the total capsule weight and
- consists, based on its weight, of at least 50 wt.%, preferably at least 60 wt.%, more preferably at least 70 wt.% and in particular at least 75 wt.% alditols, preferably mannitol.

6. The water-in-oil emulsion according to one of claims 1 to 5, **characterized in that** it contains multilayer pigment capsules which contain, based on the weight of the pigment capsule,
from 10 to 40 wt.%, preferably from 12.5 to 37.5 wt.%, more preferably from 15 to 35 wt.%, even more preferably from 17.5 to 32.5 wt.%, particularly preferably from 20 to 30 wt.% and in particular from 22.5 to 27.5 wt.% mannitol.

7. The water-in-oil emulsion according to one of claims 1 to 6, **characterized in that** the first shell surrounding the particle core
- makes up from 20 to 40 wt.% of the total capsule weight and
- consists, based on its weight, of at least 50 wt.%, preferably at least 60 wt.%, more preferably at least 70 wt.% and in particular at least 75 wt.% one or more of the pigments Pigment Green (CI 10006 green), Pigment Brown 1 (CI 12480 brown), Chlorinated Phthalocyanine (CI 74260 green), Pigment Red 101 and 102 (CI 77015 red), Carbon (CI 77266 black), Pigment Black 9 (CI 77267 black), Pigment Blue 28, Pigment Green 14 (CI 77346 green), Pigment Metal 2 (CI 77400 brown), Gold (CI 77480 brown), Iron Oxide and Hydroxide (CI 77489 orange), Iron Oxide (CI 77491 red), Iron Oxide (CI 77499 black).

8. The water-in-oil emulsion according to one of claims 1 to 7, **characterized in that** it contains multilayer pigment capsules which contain, based on the weight of the pigment capsule, from 10 to 40 wt.%, preferably from 12.5 to 38 wt.%, more preferably from 15 to 36 wt.%, even more preferably from 17.5 to 34 wt.%, particularly preferably from 20 to 32 wt.% and in particular from 22.5 to 30 wt.% one or more of the pigments Pigment Green (CI 10006 green), Pigment Brown 1 (CI 12480 brown), Chlorinated Phthalocyanine (CI 74260 green), Pigment Red 101 and 102 (CI 77015 red), Carbon (CI 77266 black), Pigment Black 9 (CI 77267 black), Pigment Blue 28, Pigment Green 14 (CI 77346 green), Pigment Metal 2 (CI 77400 brown), Gold (CI 77480 brown), Iron Oxide and Hydroxide (CI 77489 orange), Iron Oxide (CI 77491 red), Iron Oxide (CI 77499 black).

9. The water-in-oil emulsion according to one of claims 1 to 8, **characterized in that** the second shell surrounding the first shell
- makes up from 20 to 40 wt.% of the total capsule weight and
- consists, based on its weight, of at least 50 wt.%, preferably at least 70 wt.%, more preferably at least 85 wt.% and in particular at least 95 wt.% Titanium Dioxide (CI 77891).

10. The water-in-oil emulsion according to one of claims 1 to 9, **characterized in that** it contains multilayer pigment capsules which contain, based on the weight of the pigment capsule,
- from 22.5 to 27.5 wt.% mannitol and
- from 10 to 40 wt.%, preferably from 12.5 to 38 wt.%, more preferably from 15 to 36 wt.%, even more preferably from 17.5 to 34 wt.%, particularly preferably from 20 to 32 wt.% and in particular from 22.5 to 30 wt.% one or more of the pigments Pigment Green (CI 10006 green), Pigment Brown 1 (CI 12480 brown), Chlorinated Phthalocyanine (CI 74260 green), Pigment Red 101 and 102 (CI 77015 red), Carbon (CI 77266 black), Pigment Black 9 (CI 77267 black), Pigment Blue 28, Pigment Green 14 (CI 77346 green), Pigment Metal 2 (CI 77400 brown), Gold (CI 77480 brown), Iron Oxide and Hydroxide (CI 77489 orange), Iron Oxide (CI 77491 red), Iron Oxide (CI 77499 black).

11. The water-in-oil emulsion according to one of claims 1 to 10, **characterized in that** the third shell surrounding the second shell
- makes up from 2 to 7 wt.% of the total capsule weight and
- consists, based on its weight, of at least 50 wt.%, preferably at least 70 wt.%, more preferably at least 85 wt.% and in particular at least 95 wt.% polymethylacrylate.

12. The water-in-oil emulsion according to one of claims 1 to 11, **characterized in that** it contains multilayer pigment capsules which contain, based on the weight of the pigment capsule, from 10 to 40 wt.%, preferably from 12.5 to 38 wt.%, more preferably from 15 to 36 wt.%, even more preferably from 17.5 to 34 wt.%, particularly preferably from 20 to 32 wt.% and in particular from 22.5 to 30 wt.% Titanium Dioxide (CI 77891).

13. The water-in-oil emulsion according to one of claims 1 to 12, **characterized in that** it contains multilayer pigment capsules which contain, based on the weight of the pigment capsule,
- from 22.5 to 27.5 wt.% mannitol and
- from 10 to 40 wt.%, preferably from 12.5 to 38 wt.%, more preferably from 15 to 36 wt.%, even more preferably from 17.5 to 34 wt.%, particularly preferably from 20 to 32 wt.% and in particular from 22.5 to 30 wt.% one or more of the pigments Pigment Green (CI 10006 green), Pigment Brown 1 (CI 12480 brown), Chlorinated Phthalocyanine (CI 74260 green), Pigment Red 101 and 102 (CI 77015 red), Carbon (CI 77266 black), Pigment Black 9 (CI 77267 black), Pigment Blue 28, Pigment Green 14 (CI 77346 green), Pigment Metal 2 (CI 77400 brown), Gold (CI 77480 brown), Iron Oxide and Hydroxide (CI 77489 orange), Iron Oxide (CI 77491 red), Iron Oxide (CI 77499 black) and
- from 10 to 40 wt.%, preferably from 12.5 to 38 wt.%, more preferably from 15 to 36 wt.%, even more preferably from 17.5 to 34 wt.%, particularly preferably from 20 to 32 wt.% and in particular from 22.5 to 30 wt.% Titanium Dioxide (CI 77891).

14. The water-in-oil emulsion according to one of claims 1 to 13, **characterized in that** at least 90 wt.% of the multilayer pigment capsules have a particle size of from 75 nm to 211 µm (from 70 to 200 mesh).

## Revendications

1. Émulsion eau dans huile, contenant, par rapport à son poids total,
a) 0,1 à 5 % en poids de polyglycéryl-2-polyhydroxystéarate ;
b) au moins un composant lipidique ou cireux avec un point de fusion > 50°C,
c) au moins une huile, fluide dans des conditions normales (20° C et 1013,25 mbar) et qui ne constitue ni une substance aromatique ni une huile essentielle,
d) 5 à 70 % en poids d'eau, par rapport à la composition totale,
e) au moins une capsule de pigments multicouche, comprenant
i) un noyau de particule,
ii) une première couche englobant ce noyau de particule, qui - par rapport à son poids - comprend au moins 50% en poids de pigments colorés,
iii) une deuxième couche englobant la première couche, qui, par rapport à son poids, comprend au moins 50 % en poids de dioxyde de titane,
iv) une troisième couche qui englobe la deuxième couche et qui contient un polymère insoluble dans l'eau et thermoplastique.

2. Emulsion eau dans huile selon la revendication 1, **caractérisée en ce qu'**elle contient, par rapport à son poids, 0,25 à 4 % en poids, de préférence 0,5 à 3 % en poids, de manière davantage préférée 0,75 à 2 % en poids et en particulier 1 à 1,5 % en poids de polyglycéryl-2-dipolyhydroxystéarate.

3. Émulsion eau dans huile selon une des revendications 1 ou 2, **caractérisée en ce qu'**elle contient, en plus, 0,1 à 5 % en poids, de préférence 0,25 à 4 % en poids, de manière davantage préférée 0,5 à 3 % en poids, de manière encore davantage préférée 0,75 à 2 % en poids et en particulier 0,9 à 1,5 % en poids, de monooléate de glycérine, par rapport à son poids respectivement.

4. Émulsion eau dans huile selon l'une quelconque des revendications 1 à 3, **caractérisée en ce qu'**elle contient, par rapport à son poids, 0,25 à 5 % en poids, de préférence 0,5 à 4 % en poids, de manière davantage préférée 0,75 à 3 % du poids et en particulier 1 à 2 % en poids de stéarate de zinc.

5. Émulsion eau dans huile selon l'une quelconque des revendications 1 à 4, **caractérisée en ce que** le noyau de particule de la capsule de pigments multicouche
- constitue 20 à 40 % en poids du poids total de la capsule et
- est constitué à au moins 50 % en poids, préférentiellement à au moins 60 % en poids, tout préférentiellement à au moins 70 % en poids et particulièrement préférentiellement à au moins 75 % en poids de son poids d'alditols, de préférence de mannitol.

6. Émulsion eau dans huile selon l'une quelconque des revendications 1 à 5, **caractérisée en ce qu'**elle contient des capsules de pigments multicouches, qui, par rapport au poids de la capsule de pigments, contiennent
10 à 40 % en poids, de préférence 12,5 à 37,5 % en poids, préférentiellement 15 à 35 % en poids, tout préférentiellement 17,5 à 32,5 % en poids, particulièrement préférentiellement 20 à 30 % en poids et en particulier 22,5 à 27,5 % en poids de mannitol.

7. Émulsion eau dans huile selon l'une quelconque des revendications 1 à 6, **caractérisée en ce que** la première couche entourant le noyau de particule
- constitue 20 à 40 % en poids du poids total de la capsule et
- est constitué à au moins 50 % en poids, préférentiellement à au moins 60 % en poids, tout préférentiellement à au moins 70 % en poids et en particulier à au moins 75 % en poids, de son poids, d'un ou plusieurs des pigments Pigment Green (CI 10006 vert), Pigment Brown 1 (CI 12480 brun), phtalocyanine chlorée (CI 74260 vert), Pigment Red 101 et 102 (CI 77015 rouge), carbone (CI 77266 noir), Pigment Black 9 (CI 77267 noir), Pigment Blue 28, Pigment Green 14 (CI 77346 vert), Pigment Metal 2 (CI 77400 brun), Or (CI 77480 brun), oxydes et hydroxydes de fer (CI 77489 Orange), oxyde de fer (CI 77491 rouge), oxyde de fer (CI 77499 noir).

8. Émulsion eau dans huile selon l'une quelconque des revendications 1 à 7, **caractérisée en ce qu'**elle contient des capsules de pigments multicouches, qui contiennent, par rapport au poids de la capsule de pigments, 10 à 40 % en poids, préférentiellement 12,5 à 38 % en poids, tout préférentiellement 15 à 36 % en poids, encore plus préférentiellement 17,5% à 34 % en poids, particulièrement préférentiellement 20 à 32 % en poids et en particulier 22,5 % à 30 % en poids d'un ou plusieurs des pigments Pigment Green (CI 10006 vert), Pigment Brown 1 (CI 12480 brun), phtalocyanine chlorée (CI 74260 vert), Pigment Red 101 et 102 (CI 77015 rouge), carbone (CI 77266 noir), Pigment Black 9 (CI 77267 noir), Pigment Blue 28, Pigment Green 14 (CI 77346 vert), Pigment Metal 2 (CI 77400 brun), Or (CI 77480 brun), oxydes et hydroxydes de fer (CI 77489 orange), oxyde de fer (CI 77491 rouge), oxyde de fer (CI 77499 noir).

9. Émulsion eau dans huile selon l'une quelconque des revendications 1 à 8, **caractérisée en ce que** la deuxième couche entourant la première couche
- constitue 20 à 40 % en poids du poids total de la capsule et
- est constituée au moins à 50 % en poids, préférentiellementà au moins 70% en poids, tout préférentiellementà au moins 85 % en poids et en particulier à au moins 95 % en poids, de son poids, de dioxyde de titane (CI 77891).

10. Émulsion eau dans huile selon l'une quelconque des revendications 1 à 9, **caractérisée en ce qu'**elle contient des capsules de pigments multicouches, qui contiennent, par rapport au poids de la capsule de pigments,
- 22,5 à 27,5 % en poids de mannitol et
- 10 à 40 % en poids, préférentiellement12,5 à 38 % en poids, tout préférentiellement 15 à 36 % en poids, encore plus préférentiellement 17,5 % à 34% en poids, particulièrement préférentiellement 20 à 32 % en poids et en particulier 22,5 % à 30 % en poids d'un ou plusieurs des pigments Pigment Green (CI 10006 vert), Pigment Brown 1 (CI 12480 brun), phtalocyanine chlorée (CI 74260 vert), Pigment Red 101 et 102 (CI 77015 rouge), carbone (CI 77266 noir), Pigment Black 9 (CI 77267 noir), Pigment Blue 28, Pigment Green 14 (CI 77346 vert), Pigment Metal 2 (CI 77400 brun), Or (CI 77480 brun), oxydes et hydroxydes de fer (CI 77489 orange), oxyde de fer (CI 77491 rouge), oxyde de fer (CI 77499 noir).

11. Émulsion eau dans huile selon l'une quelconque des revendications 1 à 10, **caractérisée en ce que** la troisième couche entourant la deuxième couche
- constitue 2 à 7 % en poids du poids total de la capsule et
- est constituée au moins à 50 % en poids, préférentiellement à au moins 70% en poids, tout préférentiellement à au moins 85 % en poids et en particulier à au moins 95 % en poids, de son poids, de polyméthacrylate de méthyle.

12. Émulsion eau dans huile, selon l'une quelconque des revendications 1 à 11, **caractérisée en ce qu'**elle contient des capsules de pigments multicouches, qui, par rapport au poids de la capsule de pigments, contiennent 10 à 40 % en poids, préférentiellement12,5 à 38 % en poids, tout préférentiellement 15 à 36 % en poids, encore plus préférentiellement 17,5% à 34 % en poids, particulièrement préférentiellement 20 à 32 % en poids et en particulier 22,5 % à 30 % en poids de dioxyde de titane (CI 77891).

13. Émulsion eau dans huile selon l'une quelconque des revendications 1 à 12, **caractérisée en ce qu'**elle contient des capsules à pigments multicouches, qui contiennent, par rapport au poids de la capsule à pigments,
- 22,5 à 27,5 % en poids de mannitol et
- 10 à 40 % en poids, préférentiellement 12,5 à 38 % en poids, tout préférentiellement 15 à 36 % en poids, encore plus préférentiellement 17,5% à 34% en poids, particulièrement préférentiellement 20 à 32 % en poids et en particulier 22,5 % à 30 % en poids d'un ou plusieurs des pigments Pigment Green (CI 10006 vert), Pigment Brown 1 (CI 12480 brun), phtalocyanine chlorée (CI 74260 vert), Pigment Red 101 et 102 (CI 77015 rouge), carbone (CI 77266 noir), Pigment Black 9 (CI 77267 noir), Pigment Blue 28, Pigment Green 14 (CI 77346 vert), Pigment Metal 2 (CI 77400 brun), Or (CI 77480 brun), oxydes et hydroxydes de fer (CI 77489 orange), oxyde de fer (CI 77491 rouge), oxyde de fer (CI 77499 noir) et
- 10 à 40 % en poids, préférentiellement 12,5 à 38 % en poids, tout préférentiellement 15 à 36 % en poids, encore plus préférentiellement 17,5 à 34% en poids, particulièrement préférentiellement 20 à 32 % en poids et en particulier 22,5 à 30 % en poids de dioxyde de titane (CI 77891).

14. Émulsion eau dans huile selon l'une quelconque des revendications 1 à 13, **caractérisée en ce qu'**au moins 90 % en poids des capsules de pigments multicouches présentent une taille de particule de 75 nm à 211 pm (70 à 200 Mesh).
